Europäisches Patentamt

European Patent Office

Office européen des brevets

(19)

(11) Numéro de publication: **0 093 457**

**A2**

(12) **DEMANDE DE BREVET EUROPEEN**

(21) Numéro de dépôt: **83104934.1**

(22) Date de dépôt: **27.05.81**

(51) Int. Cl.³: **C 12 Q 1/34**
**G 01 N 33/50**

(30) Priorité: **27.05.80 FR 8011728**

(43) Date de publication de la demande:
**09.11.83 Bulletin 83/45**

(84) Etats contractants désignés:
**BE CH DE FR GB LI**

(60) Numéro de publication de la demande initiale
en application de l'article 76 CBE: **0 041 029**

(71) Demandeur: **INSTITUT PASTEUR**
**28, rue du Docteur Roux**
**F-75715 Paris Cedex 15(FR)**

(72) Inventeur: **Hosli, Peter**
**159, rue Blomet**
**F-75015 Paris(FR)**

(72) Inventeur: **Vogt, Esther**
**159, rue Blomet**
**F-75015 Paris(FR)**

(74) Mandataire: **Gutmann, Ernest et al,**
**Cabinet Plasseraud 84, rue d'Amsterdam**
**F-75009 Paris(FR)**

(54) **Procédé et compositions pour le diagnostic in vitro des maladies dues à des déficiences lysozomiales, à des aberrations chromosomiques, à la dystrophie musculaire ou à la fibrose kystique.**

(57) Procédé de diagnostic *in vitro* de la potentialité ou de l'existence de la fibrose kystique ou de la dystrophie musculaire. Il consiste à mettre en contact une culture de cellules provenant d'un individu ou d'un embryon étudié avec une composition d'induction contenant en association de l'héparine et des globulines, notamment des γ-globulines humaines, à doser, dans le milieu intracellulaire ou extracellulaire au contact duquel se trouvaient lesdites cellules, le niveau d'activité de l'une des enzymes, notamment hydrolases, qui sont produites en excès chez les individus atteints de l'une des susdites maladies et, en cas de réponse positive, à se placer dans des conditions de température pour lesquelles les hydrolases induites subissent une cinétique d'inactivation qui, lorsqu'elle se produit, peut être corrélée à la fibrose kystique et qui, lorsqu'elle ne se produit sensiblement pas, peut être corrélée à une dystrophie musculaire.

EP 0 093 457 A2

PROCEDE ET COMPOSITIONS POUR LE DIAGNOSTIC *IN VITRO DES*
MALADIES DUES A DES DEFICIENCES LYSOZOMIALES, A DES
ABERRATIONS CHROMOSOMIQUES,   A LA DYSTROPHIE MUSCULAIRE
OU A LA FIBROSE KYSTIQUE.

L'invention concerne un procédé de diagnostic *in
vitro* de maladies trouvant leur origine dans des anomalies
lysozomiales (ou lysozomales), la fibrose kystique, la dystrophie musculaire et/ou les aberrations chromosomiques, un
procédé dérivé du précédent permettant la détermination *in
vitro* de la possibilité d'action de médicaments à l'étude
sur ces maladies, ainsi que des réactifs susceptibles
d'être mis en oeuvre dans ces procédés.

Il est à ce jour pratiquement établi que de nombreuses maladies lysozomiales trouvent leur origine dans une
modification  d'origine génétique des équilibres enzymatiques
au sein des cellules de l'organisme.

A titre d'un premier type d'exemple de ces maladies
lysozomales, on peut citer les maladies classiques dites de
stockage ou d'accumulation dues à l'incapacité des cellules
de l'hôte de synthétiser des enzymes déterminées aptes à métaboliser ou cataboliser des substrats déterminés, plus particulièrement certains types d'hydrolases. Il est ainsi
connu par exemple que la maladie de Pompe est due à une déficience en
$\alpha$-glucosidase, d'où l'impossibilité pour les cellules atteintes de digérer ou d'hydrolyser le substrat correspondant, le
glycogène, lequel tend alors à s'accumuler dans l'organisme
de l'hôte. Il en est encore de même des maladies du type
$gM_1$-gangliosidose, qui sont dues à une déficience en
$\beta$-galactosidase. Les cellules de l'individu affectées ne sont
alors pas aptes à hydrolyser les $gM_1$-gangliosides .

Une autre catégorie de maladies lysozomiales se
manifeste au contraire par des libérations d'hydrolases dans
les espaces extracellulaires et par conséquent en des déficiences intracellulaires multiples en ces hydrolases.

L'hypothèse a été formulée que ces libérations incontrôlées trouvaient leur origine dans un défaut génétique
affectant certains marqueurs portés par ces hydrolases, marqueurs qui servent normalement à ancrer ces hydrolases sur
des récepteurs de membranes lysozomaux spécifiques. Ces marqueurs paraissent devoir être constitués par des oligosaccharides phosphorylés fixés sur les protéines enzymatiques à l'occasion d'un processus secondaire intervenant lors de leur passage à travers les cavités du réticulum endoplasmique.

La conséquence de modifications du système lysozomal est là encore une accumulation dans l'organisme de différents substrats macromoléculaires non hydrolysés. Un certain nombre de mucolipidoses se rattachent à cette catégorie de maladies. Il semble ainsi établi que la maladie des "cellules-I" ("I-cell disease") est due à une phosphorylation anormale d'un groupe mannose, d'où des libérations incontrôlées d'hydrolases dans les espaces extracellulaires ou - lorsque ces cellules sont cultivées *in vitro* - dans le milieu de culture.

A cette catégorie de maladies se rattache la fibrose kystique (souvent visée ci-après par les initiales "CF"), également dénommée mucoviscidose, qui est une maladie génétique récessive autosomale extrêmement grave, particulièrement fréquente dans les populations d'origine européenne. En France un nouveau-né sur 2.000 à 2.500 en est affecté. Cette maladie se manifeste en général aussitôt après la naissance. Les syndromes les plus caractéristiques consistent dans le développement d'obstructions pulmonaires chroniques, d'insuffisances pancréatiques, un défaut de métabolisation des graisses et une sensibilité extrême aux infections, notamment au niveau des régions respiratoires. La mort s'ensuit habituellement au cours des toutes premières années de la vie de l'enfant. Au niveau cellulaire, on constate des déficiences intracellulaires partielles multiples en hydrolases, des fuites excessives de ces mêmes hydrolases dans les fluides extracellulaires (plasma sanguin, milieux de cultures). Certaines des enzymes concernées sont encore affectées d'une thermolabilité, qui peut être mise à profit pour un diagnostic différentiel *in vitro* de la fibrose kystique.

A l'inverse, ce sont, dans une autre catégorie de maladies, les récepteurs lysozomiaux de membranes eux-mêmes qui peuvent être défectueux (maladie de Chediak-Higashi), d'où encore la conséquence d'une libération incontrôlée d'hydrolases dans le milieu extracellulaire et la métabolisation insuffisante, sinon inexistante de divers substrats.

De nombreuses autres maladies, plus particulièrement celles dues à des anomalies chromosomiques, se manifestent par des modifications au niveau de la synthèse des enzymes qui s'avèrent pouvoir être dépistées au niveau cellulaire. Il a été par ailleurs constaté, dans le cadre de la présente invention, qu'une maladie mettant en jeu une anomalie au niveau du chromosome X (mutation ponctuelle sur ce chromosome) et qui est particulièrement répandue - la dystrophie musculaire -, peut se manifester dans les conditions qui seront décrites plus loin par une libération dans les milieux extracellulaires d'enzymes spécifiques. Il a cependant été constaté qu'elles ne présentent pas de thermolabilité différentielle vis-à-vis des enzymes correspondantes produites par les cellules d'individus sains. Plus généralement, on constate que les cultures de cellules présentant des anomalies chromosomiques sont susceptibles d'engendrer , dans les conditions qui seront décrites plus loin, des variations importantes vis-à-vis de la normale des quantités d'enzymes, notamment d'hydrolases, produites par rapport aux cellules normales. Ce phénomène se concrétise par des excédents intracellulaires, extracellulaires ou les deux à la fois, d'enzymes hydrolytiques.

En raison du caractère particulièrement grave de la plupart de ces maladies, il est souhaitable de pouvoir dépister ces anomalies génétiques aussitôt que possible. Les chances de survie d'un nouveau-né potentiellement affecté de l'une de ces maladies seront d'autant meilleures qu'il pourra sans tarder être soumis à une thérapie intensive. Il serait mieux encore de dépister cette anomalie dans la phase néonatale, éventuellement même dans la toute première phase de la grossesse, à un moment où une interruption de grossesse thérapeutique pourrait éventuellement encore être indiquée.

Certes, il existe déjà à ce jour des techniques permettant d'apprécier sur des prélèvements de liquide amniotique des anomalies chromosomiques, lorsqu'elles se manifestent par des modifications d'ordre structurel. On conçoit cependant la difficulté d'effectuer des caryotypes systématiques sur un grand nombre de prélèvements cellulaires. Les techniques de dépistage s'avèrent cependant encore plus difficiles à ce jour lorsque les anomalies en question sont invisibles à l'oeil et sont l'expression d'erreurs futures de contrôle ou de régulation du métabolisme cellulaire. Les procédés proposés jusqu'à ce jour pour les dépistages néonataux n'ont donc guère été développés, tant en raison des résultats ambigus auxquels ils conduisent que de la difficulté technique de leur mise en oeuvre.

Il serait encore mieux de pouvoir évaluer préventivement les risques de transmission d'un défaut génétique par des parents apparemment sains à leur descendance. A titre d'exemple, on peut mentionner que certaines de ces maladies, telles que la mucoviscidose, sont des affections familiales se transmettant selon le mode autosomal récessif. Il semble à ce jour établi que sont susceptibles d'être affectés de la mucoviscidose les enfants dont le patrimoine génétique résulte de la recombinaison chromosomique au moment de la fécondation des patrimoines génétiques du père et de la mère, lorsque tous deux sont porteurs d'une mutation spécifique, sans pour autant être eux-mêmes affectés de cette maladie. Ces parents seront ci-après dits "CF-hétérozygotes". Bien entendu, cette expression s'étend également à leurs cellules et, d'une façon générale, à leur patrimoine génétique propre. Sont donc susceptibles d'être affectés de la maladie, les enfants qui reçoivent en partage ces défauts génétiques de leurs deux parents , eux-mêmes étant dits par la suite "CF-homozygotes". Par extension, on utilisera aussi ces expressions "hétérozygotes" et "homozygotes", à l'égard de parents et enfants respectivement porteurs d'une anomalie génétique quelconque.

L'invention a pour but de remédier aux inconvénients susmentionnés, plus particulièrement de fournir un procédé permettant tant le dépistage global *in vitro* chez un individu de l'existence éventuelle ou potentielle de l'une quelconque d'une pluralité de maladies prédéterminées du genre en question, que le diagnostic à la fois plus sûr et plus aisé qu'il n'était possible jusqu'à ce jour de l'existence éventuelle ou potentielle de l'une d'entre elles.

Le procédé selon l'invention a encore pour but la réalisation du test de dépistage, non seulement chez les nouveau-nés ou d'une façon plus générale les enfants, mais aussi chez l'embryon et de préférence même dans les toutes premières semaines de la grossesse.

La demande de brevet n° 79 14234 déposée le 1er juin 1979 au nom du demandeur décrivait pour la fibrose kystique l'obtention d'un procédé permettant le dépistage systématique au niveau des populations de ceux des individus éventuellement porteurs d'un défaut génétique "hétérozygote". Un tel test de diagnostic systématique peut, par exemple, être mis en oeuvre à l'occasion des visites médicales prénuptiales de plus en plus couramment prescrites par les législations nationales.

La présente invention vise l'obtention d'un procédé permettant de tester la valeur potentielle d'un médicament à l'étude contre une ou plusieurs des maladies du genre en question.

L'invention met à profit un certain nombre de constatations et d'hypothèses qui s'appuient sur un support expérimental étendu concernant la régulation génétique. C'est ainsi que l'on peut à ce jour penser que le signal primaire susceptible d'activer les gènes qui codent pour la synthèse des différentes hydrolases nécessaires à la digestion intracellulaire des espèces macromoléculaires correspondantes est induit par ces espèces macromoléculaires elles-mêmes, telles que divers polysaccharides, le glycogène, les gangliosides, etc., lorsque celles-ci entrent dans les cellules. Ce signal serait normalement faible et de courte durée dans des cellules qui sont pourvues d'un équipement enzymatique normal, qui est alors capable d'hydrolyser lesdites macromolécules. Il serait cependant beaucoup plus fort et de longue durée dans des cellules qui ne sont pas capables de digérer l'une au moins desdites macromolécules. Il semble que l'acide adénylique cyclique (AMP cyclique ou

6    **0093457**

cAMP) intervient dans ces processus en tant que messager hormonal secondaire, en tant qu'il coordonne l'induction des hydrolases par activation des gènes correspondants, lorsque s'accroît sa concentration cellulaire.

C'est l'ensemble de ces phénomènes physiologiques qu'exploite l'invention en vue du diagnostic *in vitro* de l'existence éventuelle ou potentielle d'anomalies lysozomiales, de fibrose kystique, de la dystrophie musculaire et des anomalies chromosomiques ; plus particulièrement :

a) des maladies génétiques du dispositif de digestion intracellulaire, notamment les maladies lysozomiales classiques d'accumulation, le syndrome de Chédiak-Higashi, la fibrose cystique,la dystrophie musculaire de Duchenne, etc..

b) des maladies génétiques mettant en jeu sous l'effet de stimuli hormonaux des réactions excessives accompagnées d'une induction coordonnée d'hydrolases dans les cellules affectées d'aberrations chromosomiques, telles que trisomies, monosomies, trisomies partielles (duplications), monosomies partielles (délétions), etc..

Le procédé selon l'invention est, dans l'une de ses premières variantes, caractérisé par la mise en contact d'une culture de cellules de l'hôte avec un "cocktail" contenant les réactifs suivants :

- 1) au moins un inducteur d'au moins une enzyme, plus particulièrement d'une hydrolase corrélable à une maladie déterminée et dont le comportement doit être apprécié, ou alternativement une hormone stimulant l'adénylcyclase, ou encore les deux à la fois ;

- 2) un inhibiteur des phosphodiestérases, du type de celles qui peuvent dégrader l'AMP cyclique, tel que la théophylline ;

- 3) un inhibiteur de la synthèse des protéines ; les concentrations relatives de ces constituants dans le milieu de culture étant réglées de façon à permettre un comportement nettement différencié de cellules saines et de cellules porteuses de l'anomalie recherchée (notamment dans le cas de cellules homozygotes), à ne pas affecter sensiblement le développement de cellules saines, mais à entraîner l'interruption du développement, voire la
mort desdites cellules homozygotes ou hétérozygotes.

On appréciera que ce cocktail de réactifs - qui fait
également partie de l'invention en tant que tel - semble
avoir pour effets combinés, d'une part, une induction d'un
signal primaire renforcé et de longue durée, lorsque la
cellule est affectée par fibrose kystique, dystrophie musculaire ou une anomalie lysozomiale, une aberration chromosomique, d'autre part, la stabilisation d'un signal secondaire (par hypothèse celui de l'AMP cyclique) également
renforcé du fait de l'inhibition des phosphodiestérases qui
tendent normalement à la dégradation de l'AMP cyclique en
adénosine 5'-monophosphate et, d'autre part encore, l'incapacité
de la cellule de répondre à ces stimuli , faute de pouvoir
synthétiser des protéines sous l'action de l'inhibiteur de
la synthèse des protéines.

A titre d'exemple l'inducteur est constitué par au
moins l'un de ceux qui sont propres à normalement induire la
modification de la synthèse enzymatique dans la maladie à étudier. Il
s'agit par exemple des macromolécules naturelles que les cellules -
lorsqu'elles sont affectées par la maladie en cause - ne
peuvent pas cataboliser (par exemple le glycogène dans le
cas de la maladie de Pompe, des gangliosides, notamment
des $gM_1$-gangliosides dans le cas des maladies du type gangliosidoses, des glycoprotéines ou mucopolysaccharides,
dans le cas de celles des autres maladies qui sont attribuées à l'incapacité des cellules de l'hôte à cataboliser
ces substrats, etc.). Les inducteurs du genre en question
peuvent également être différents des substrats naturels
correspondant à la maladie étudiée, pour autant qu'ils
soient capables d'induire des effets semblables au niveau
des cellules affectées par les maladies en question. A titre
d'exemple particulièrement avantageux d'inducteurs naturels,
on citera le mélange héparine-immunoglobulines, celles-ci
étant de préférence d'origine humaine, et notamment constituées par des γ-globulines humaines, ce mélange étant -
conformément à un aspect particulier de l'invention -
propre à servir à la caractérisation de cellules affectées
par l'anomalie caractéristique de la fibrose kystique
(cellules homozygotes ou hétérozygotes) ou de la dystrophie musculaire (cellules homozygotes).

A titre d'exemples préférés d'hormones stimulant l'adénylcyclase, on mentionnera essentiellement les prostaglandines, notamment les prostaglandines $E_1$, ou mieux encore les prostaglandines $E_2$.

En ce qui concerne l'inhibiteur des phosphodiestérases entrant dans le cocktail de l'invention, on peut naturellement remplacer la théophylline par toute autre substance ayant une action semblable. On peut citer à titre d'exemple la 4-(4,3-diméthoxybenzyl)-2-imidazoline ou encore ceux mentionnés dans                l'article intitulé "Inhibitors and Activators of Cyclic Nucleotide Phosphodiestérases" de M. Chasin et D.N. Harris, dans le 7ème tome de l'ouvrage intitulé "Advances in Cyclic Nucléotide Research" de Greengard et Robinson, Raven Press, 1976.

En ce qui concerne enfin l'inhibiteur de la synthèse des protéines on peut avoir recours à tout composé actif à une étape quelconque de la synthèse protéique, par exemple d'un inhibiteur agissant au niveau de l'activation du gène, tel que l'actinomycine D, ou d'un inhibiteur direct de la synthèse des protéines, tel que l'émétine, notamment le dichlorhydrate d'émétine, ou, de préférence, le cycloheximide.

Dans ce qui précède, on a essentiellement considéré le cas d'un cocktail pouvant ne contenir qu'un seul inducteur caractéristique d'une anomalie recherchée, ou seulement une hormone stimulatrice de l'adénylcyclase. Il est cependant particulièrement intéressant d'avoir recours à un cocktail comprenant un grand nombre de ces inducteurs et, le cas échéant en outre, l'hormone stimulante de l'adénylcyclase, d'où la possibilité de réaliser des "dépistages globaux" de l'existence éventuelle de l'une quelconque des différentes maladies auxquelles correspondent respectivement les différents inducteurs du cocktail en question et, le cas échéant en outre, de toute aberration chromosomique révélable par l'hormone stimulante de l'adénylcyclase.

Dans cette dernière hypothèse, les épreuves de diagnostics pourront comporter plusieurs phases, par exemple :

- une première phase dans laquelle sera déterminée l'existence ou la potentialité d'une maladie génétique déterminée à l'aide d'un tel "cocktail global" (celui-ci ne permettra cependant pas d'identifier de façon immédiate la maladie en cause) ;

- une ou plusieurs phases supplémentaires mettant en jeu des sous-cocktails, notamment ne contenant, au titre du premier (ou des premiers) des constituants définis plus haut de façon générale, que des inducteurs de maladies lysozomiales, à l'exclusion de l'hormone stimulant l'adénylcyclase, ou vice versa.

Une réponse positive au sous-cocktail ne contenant que des inducteurs des maladies lysozomales incitera le spécialiste à répéter l'expérience de culture des cellules originaires du même hôte, mais avec des cocktails spécifiques, ne contenant qu'un seul inducteur, lui-même spécifique d'une ou d'un nombre réduit de maladies, quitte à achever l'identification de la maladie spécifique par d'autres méthodes analytiques .

Au contraire, une réponse positive avec le cocktail ne contenant que l'hormone stimulatrice de l'adénylcyclase, à l'exclusion de tout inducteur de maladie lysozomiale, incitera le chercheur à rechercher la nature de l'anomalie chromosomique alors révélée elle-même dans sa globalité, par toute autre méthode connue en soi, telle qu'une technique d'analyse de structure des chromosomes susceptibles d'être affectés.

A titre d'exemple de "cocktail global", on peut mentionner ceux contenant, en sus de l'inhibiteur de phosphodiestérases, notamment la théophylline, et de l'inhibiteur de la synthèse de protéines, notamment le cycloheximide :

- une prostaglandine, notamment une prostaglandine $E_2$,
- de l'héparine, des immunoglobulines, du glycogène, les différents gangliosides mis en jeu dans les différentes gangliosidoses, les différents mucopolysaccharides mis en jeu dans les mucopolysaccharidoses, etc..

Les cocktails plus spécifiques ou sous-cocktails ne contiendront plus alors, en sus de la théophylline et du cycloheximide, que des groupes réduits de ces inducteurs, voire un seul de ces inducteurs.                          **0093457**

L'invention concerne plus particulièrement des cocktails dans lesquels sont associés à la théophylline et au cyclohéximide (ou autres composés ayant des fonctions analogues), d'une part, une prostaglandine, notamment une prostaglandine $E_1$, ou de préférence une prostaglandine $E_2$, et, d'autre part, de l'héparine et une globuline, notamment une γ-globuline humaine.

L'association héparine-globuline constitue elle-même un inducteur particulier conforme à l'invention, apte à permettre le diagnostic *in vitro* de la présence ou de la potentialité d'une fibrose kystique ou d'une dystrophie musculaire. Il a en effet été constaté, conformément à l'invention, que la mise en présence de cellules affectées de l'anomalie correspondant à la dystrophie musculaire avec l'association héparine-globuline avait pour effet l'induction d'une libération d'hydrolases par lesdites cellules.

Le susdit cocktail permettra donc la détection de la présence soit d'une anomalie chromosomique, soit d'une fibrose kystique, soit d'une dystrophie musculaire.

Avantageusement, les proportions des divers constituants dans le cocktail de l'invention seront telles qu'après mise en contact de ces cocktails avec le milieu de culture contenant les cellules à étudier, on obtienne les concentrations finales respectives suivantes (exprimées par rapport au ml de milieu de culture contenant ces constituants) :

- de 180 µg à 1000 µg, notamment de l'ordre de 600 µg par ml pour l'héparine,
- de 180 µg à 600 µg, notamment de l'ordre de 400 µg par ml pour la γ-globuline,
- de 0,8 µg à 10 µg, notamment de l'ordre de 1 µg par ml pour la prostaglandine $E_2$,
- de $10^{-5}$ à $10^{-2}$, notamment $10^{-3}$ M de théophylline,
- de 8 µg à 25 µg, notamment 10 µg par ml pour le cyclohéximide (concentration finale).

L'invention concerne également les cocktails préférés eux-mêmes, tels qu'ils ont été définis ci-dessus, contenant les différents constituants dans des proportions

**0093457**

telles qu'ils puissent donner les concentrations relatives indiquées plus haut vis-à-vis de 1 ml de milieu de culture.

L'invention concerne également les sous-cocktails du genre en question dans lesquels manquent soit l'héparine et les $\gamma$-globulines, soit la prostaglandine $E_2$. Ces deux derniers cocktails permettront par conséquent une première différenciation entre les cellules atteintes d'une anomalie chromosomique (réponse positive au cocktail à base de prostaglandine $E_2$) et des cellules atteintes d'une anomalie correspondant soit à la fibrose kystique, soit à la dystrophie musculaire. Ces dernières cellules devront alors faire l'objet d'un essai complémentaire, notamment selon d'autres méthodes de diagnostic.

Avantageusement, on mettra à profit le fait que certaines des enzymes affectées par la fibrose kystique, présentent une thermolabilité plus grande que lorsqu'elles sont originaires de cellules saines, différence qui n'existe pas dans le cas des enzymes mises en jeu dans la dystrophie musculaire.

En particulier, on peut mettre à profit, pour détecter la présence ou la potentialité d'une fibrose kystique, le fait que les α-mannosidases ou phosphatases acides produites par les cellules concernées tendent à subir une inactivation beaucoup plus rapide à 40°,5 C pour les premières, à 36°,5 C pour les secondes, lorsqu'elles proviennent de cellules d'individus "CF-homozygotes" ou "CF-hétérozygotes" que lorsqu'elles proviennent d'individus normaux. Ce même type d'inactivation totale ou partielle à ces températures n'est pas observable lorsque les cellules proviennent d'individus souffrant de dystrophies musculaires ou menacées par cette maladie, d'où par conséquent la possibilité de différencier les deux maladies dans un tel test final.

Dans la forme de mise en oeuvre la plus accessible du procédé qui a été défini ci-dessus, la différenciation entre cellules saines et cellules malades met essentiellement en jeu la capacité des cellules en question à survivre en présence des cocktails du genre en question, dans le cas de cellules saines, et à mourir au bout d'un certain temps de culture en présence de ces cocktails, comme cela sera illustré dans l'exemple plus loin, dans le cas de cellules malades.

Conformément à un développement supplémentaire de l'invention, ce test peut encore être mis en oeuvre pour déterminer la potentialité que possède un médicament déterminé d'exercer une activité favorable à l'égard de cellules malades qui ne survivent normalement pas aux conditions de mise en oeuvre du test qui vient d'être décrit. Ce développement consiste à réaliser une culture de cellules malades en présence du cocktail contenant soit l'inducteur spécifique (ou les inducteurs spécifiques) de la maladie dont sont atteintes les cellules et de doses du médicament à étudier. L'action favorable du médicament est alors susceptible de permettre la survie desdites cellules malades au bout d'un temps de culture pour lequel elles auraient autrement cessé de se développer ou seraient mortes, en l'absence du médicament en question.

L'invention fournit donc un premier procédé de "screening" ou recherche systématique du médicament susceptible d'agir au niveau cellulaire.

On décrit ci-après un exemple de mise en oeuvre pratique d'un essai mettant en jeu les cocktails et sous-cocktails préférés de l'invention, à l'égard de fibroblastes humains (type de cellules qui n'est ici pris qu'à titre d'exemple).

Les cellules à étudier, prélevées chez des patients et suspectées de présenter soit une anomalie chromosomique, soit l'une des deux maladies que sont la fibrose kystique ou la dystrophie musculaire, sont mises en culture, à 37°C, dans des boîtes de pétri ou dans des boîtes Nunclon de 24 alvéoles. Le milieu de culture est du Ham F 10 supplémenté avec 15 % de sérum de veau foetal.

16 heures plus tard, on ajoute aux cultures la composition selon l'invention dans laquelle les différents réactifs sont dans les proportions adéquates permettant l'ajustement final suivant des concentrations relatives en ses constituants essentiels vis-à-vis de 1 ml de culture :

- héparine ................ 600 µg/ml,
- γ-globuline humaine ...... 400 µg/ml,
- prostaglandine $E_2$ ........ 1 µg/ml
- théophylline ............. $10^{-3}$ M,
- cycloheximide ........... 10 µg/ml.

On laisse 3 jours à incuber à 37°C. On lave trois fois chaque boîte de pétri ou chaque alvéole avec une solution tamponnée et on ajoute ensuite 1 ml de méthanol à 70 % pour fixer les cellules. On laisse 5 minutes en contact. On sèche. On colore ensuite avec du giemsa, colorant qui ne peut colorer que des cellules vivantes.

Les alvéoles des boîtes Nunclon colorées, révèlent donc les cellules normales ; les cellules mutées, elles, n'ont pas résisté à l'action de la composition inductrice de la synthèse d'enzymes hydrolytiques.

Les alvéoles ou boîtes de pétri non colorées, dans lesquelles les cultures cellulaires ont donc été tuées, témoignent de ce qu'elles présentaient l'une des anomalies sus-mentionnées.

Des cellules provenant des mêmes patients peuvent alors être remises en culture dans les mêmes conditions en présence de sous-cocktails, identiques au cocktail global précédent, sauf qu'ils ne contiennent pas, soit la prostaglandine $E_2$, soit l'héparine et la $\gamma$-globuline humaine, de façon à procéder au premier type de différenciation décrit plus haut, entre anomalies chromosomiques de structure, d'une part, une fibrose kystique ou dystrophie musculaire, d'autre part.

Ces deux dernières maladies pourront d'ailleurs être également ensuite différenciées entre elles, notamment en ayant recours à la méthode qui a également été rappelée plus haut mettant en jeu l'existence ou non de thermolabilités de certaines des enzymes impliquées dans ces maladies.

Les tests qui viennent d'être décrits peuvent être aisément adaptés à l'étude d'un médicament susceptible d'agir au niveau cellulaire. Dans le cas où l'on souhaite par exemple étudier l'action d'une nouvelle substance vis-à-vis de la fibrose kystique, on prépare une gamme de doses croissantes du médicament, et on procède à une série de cultures des cellules connues pour être atteintes par la maladie, en présence, d'une part, du sous-cocktail spécifique à cette maladie et, d'autre part, de concentrations croissantes du médicament étudié. Le même procédé est appliqué à des cultures de cellules normales servant de témoins.

Les médicaments inactifs n'altéreront évidemment pas le résultat observé, à savoir la non-coloration finale des cultures étudiées. Par contre, les doses de médicaments aptes à au moins retarder l'effet nocif de la maladie pourront être repérées par la coloration retrouvée des cultures de cellules correspondantes par le giemsa.

Il va de soi que l'on peut utiliser, pour repérer les cellules non endommagées et les cellules endommagées, toute autre procédure ou technique que la coloration avec le giemsa (ou avec un autre colorant) pour différencier des cultures cellulaires encore vivantes et des cultures cellulaires qui ont cessé de se développer, voire même qui sont mortes.

La composition selon l'invention peut être préparée au moment de l'emploi. Elle peut aussi être préparée à l'avance, notamment se présenter sous forme d'une solution contenant :

- de 1.800 μg à 10.000 μg, notamment de 6.000 μg d'héparine par ml,
- de 1.800 μg à 6.000 μg, notamment de 4.000 μg par ml de γ-globulines humaines,
- de 8 μg à 100 μg, notamment de 10 μg par ml de prostaglandine $E_2$,
- de $10^{-4}$ à $10^{-1}$ notamment de $10\ M^{-2}$ de théophylline,
- de 80 μg à 250 μg, notamment de 100 μg par ml d'une solution à 10 % en poids de cycloheximide, notamment dans une solution tamponnée au citrate pH 5,4.

Bien entendu, l'invention concerne également les sous-cocktails contenant les mêmes substances aux mêmes concentrations, à l'exception soit de l'héparine et des γ-globulines humaines, soit de la prostaglandine $E_2$.

D'une façon générale, on pourra constituer tout autre type de cocktail inducteur, apte à permettre le dépistage d'autres types de maladies lysozomales selon le même principe, les inducteurs précédents étant remplacés par ceux correspondant à la maladie que l'on désire dépister. Ces derniers peuvent également venir s'ajouter aux précédents dans le cas où l'on souhaiterait réaliser des cocktails permettant un dépistage global en une seule fois d'un plus grand nombre de maladies. D'une façon générale, les inducteurs, notamment les substrats que les cellules atteintes desdites maladies ne sont pas capables de cataboliser, pourront être utilisés dans des gammes de concentrations généralement situées entre    300 μg à   2000 μg par ml de la composition. Leurs doses peuvent également être exprimées par leur titre final vis-à-vis du milieu de culture des cellules soumises à l'essai, notamment de 300 μg à    2000    μg par ml de milieu de culture.

Selon une seconde variante du procédé selon l'invention, on met en oeuvre, pour le diagnostic *in vitro* d'éventuelles maladies du genre en question, les susdits cocktails, cependant limités aux inducteurs  et/ou à l'hormone stimulant l'adénylcyclase, plus particulièrement à l'exclusion des inhibiteurs des phosphodiestérases et de la synthèse des protéines, et on dose les niveaux d'activité enzymatique induits par lesdits cocktails dans les cellules (milieux intracellulaires) ou dans un milieu extracellulaire, tel qu'un milieu de culture. Les niveaux d'activité mesurés s'avèrent nettement plus élevés dans les cellules malades que dans les cellules saines. Ces niveaux d'activité seront globalement plus élevés dans le cas des cellules caractérisées par une aberration chromosomique repérable par l'hormone stimulant les adénylcyclases (en raison d'une hyper-réaction de la synthèse    enzymatique de ces cellules sous l'effet de cette stimulation) ou affectées d'une maladie lysozomiale ou de la fibrose kystique ou de la dystrophie musculaire.

La lecture de l'activité enzymatique de l'enzyme de référence peut être faite par fluorescence, par colorimétrie, par dosage spécifique de l'enzyme hydrolytique choisie par l'expérimentateur (parmi toutes les enzymes hydrolytiques, certaines conviennent mieux pour le dosage intracellulaire et d'autres pour le dosage dans le milieu de culture).

Il s'est avéré que la phosphatase alcaline, qui est l'une des enzymes hydrolytiques induites après l'action d'un inducteur, est choisie lorsqu'on veut faire une mesure de l'activité enzymatique intracellulaire.

Par contre, lorsque l'on veut mesurer l'activité enzymatique dans le milieu de culture, on choisit plutôt l'hexosaminidase.

Avantageusement, le diagnostic de cellules normales ou cellules mutées est basé sur la mesure de taux de production des enzymes concernées, mettant en jeu soit des différences substantielles mesurables de niveaux d'activité enzymatique, soit des variations des propriétés de l'enzyme de référence, notamment de thermolabilité ou de cinétique d'activation ou d'inactivation de cette enzyme (par exemple cas de la fibrose kystique), selon que ces niveaux différents d'activité enzymatique ou ces propriétés différentes de l'enzyme peuvent être mis en évidence entre cellules saines et cellules atteintes de l'une des maladies recherchées.

Ces différences de propriétés prennent une importance toute particulière lorsque l'on distingue non seulement les cellules malades ou homozygotes des cellules "normales", mais aussi - dans le cas de la fibrose kystique - les cellules homozygotes des cellules hétérozygotes provenant d'individus apparemment sains.

Il a en effet été mis en évidence que les enzymes telles que l'α-mannosidase ou la phosphatase acide peuvent subir , à une température supérieure à $40°,5$ C pour la première et à $36°,5$ C pour la seconde, une inactivation plus rapide lorsqu'elles proviennent d'individus "CF-homozygotes" que lorsqu'elles proviennent d'individus "CF-hétérozygotes", la même différence s'observant à nouveau entre individus hétérozygotes et individus normaux. Ces variations de propriétés n'apparaissent pas sur les mêmes enzymes, lorsqu'elles proviennent de cellules saines ou de cellules atteintes par l'anomalie lysozomale caractéristique de la dystrophie musculaire, d'où la possibilité de différencier les cellules atteintes de fibrose kystique et celles atteintes de dystrophie musculaire.

La mise en évidence,par exemple, de la fibrose kystique ou de la dystrophie musculaire peut donc se faire directement sur culture de cellules après induction enzymatique. Bien entendu on peut avoir recours dans ce cas, comme d'ailleurs dans celui de la première variante envisagée plus haut, à toute autre méthode de diagnostic permettant de différencier entre la fibrose kystique et la dystrophie musculaire.

Bien entendu, cette variante de procédé selon l'invention peut être étendue à la détection de l'existence ou de la potentialité d'autres maladies encore, plus particulièrement des maladies lysozomiales qui ont déjà été évoquées plus haut. Dans ce cas, la susdite composition d'induction destinée à être mise en contact avec la culture de cellules à étudier contient en outre au moins un inducteur caractéristique des susdites autres maladies et ayant pour effet d'induire des niveaux d'activité excédentaires des enzymes corrélables à ces maladies dans celles des cultures de cellules originaires d'un individu qui en serait affecté.

De préférence, la proportion relative dudit inducteur est ajustée de façon à ce que la concentration finale de la culture en cet inducteur soit comprise entre 300 et 2.000 µg par ml de culture.

Comme dans le cas précédent, le diagnostic peut être fait en plusieurs étapes, en mettant en jeu des compositions ou cocktails d'induction de plus en plus spécifiques, le procédé étant alors plus particulièrement caractérisé en ce que :

- dans une première phase, on met en contact une culture desdites cellules avec une composition d'induction ou "cocktail global" contenant une pluralité des inducteurs ou groupes d'inducteurs en vue de détecter l'existence ou la potentialité de l'une des maladies ou anomalies comprises parmi le groupe de maladies auquel correspondent lesdits inducteurs ou groupes d'inducteurs et, si l'activité de l'enzyme dosée atteint un niveau attestant de la présence de l'une des susdites maladies ou anomalies,

- dans une ou plusieurs phases supplémentaires, on met en contact des cultures de cellules provenant du même individu ou embryon avec des compositions ou "sous-cocktails" plus spécifiques contenant des inducteurs ou groupes de maladies plus spécifiques, en vue de la répé-

19

tition du susdit dosage. **0093457**

L'invention est encore relative aux compositions inductrices elles-mêmes contenant des proportions relatives d'héparine et de γ-globulines, telles qu'elles permettent par mélange avec un milieu distinct, tel qu'un milieu de culture, l'obtention de concentrations relatives finales telles qu'indiquées ci-après à propos du premier test pour le diagnostic de la fibrose kystique ou de la dystrophie musculaire.

Bien entendu, elles peuvent en outre contenir d'autres inducteurs ou agents de stimulation des adénylcyclases, telles que la prostaglandine $E_2$, celle-ci se trouvant alors contenue dans la composition dont question ci-dessus, dans des proportions telles qu'elles permettent notamment l'obtention finale, dans les mêmes conditions, des intervalles de concentrations relatives en héparine, en γ-globuline et en prostaglandine $E_2$ correspondant à celles du "cocktail global" mentionné ci-après à propos du deuxième test pour le diagnostic de la fibrose kystique, de la dystrophie musculaire ou des anomalies chromosomiques.

20

Les conditions expérimentales des tes**0093457**rs avantageusement les suivantes :

1°) Pour le diagnostic de la fibrose kystique ou de la dystrophie musculaire, la composition du cocktail inducteur est ajustée, pour obtenir des concentrations finales en héparine et γ-globuline humaine, vis-à-vis de 1 ml de culture :

- de 150 à 400, notamment 200 µg par ml de milieu de culture pour l'héparine,
- de 150 à 400, notamment 200 µg par ml de milieu de culture pour la γ-globuline.

2°) Pour le diagnostic des anomalies chromosomiques, la teneur de la composition en prostaglandine $E_2$ est ajustée de façon à obtenir une concentration finale de 0,8 à 10, notamment 1 µg/ml.

Les cellules normales soumises à ce cocktail ne présentent pas une synthèse d'enzymes hydrolytiques très sensiblement différente de celle des cellules normales témoins.

Par contre, les cellules mutées présentent une augmentation du taux d'enzymes hydrolytiques que ce soit au niveau intracellulaire ou dans le milieu de culture.

Généralement, le test pour le diagnostic utilise une composition regroupant les constituants définis sous 1° et 2° ci-dessus qu'en une seule opération, on peut diagnostiquer une cellule anormale (qu'il s'agisse de la fibrose kystique, de la dystrophie musculaire ou d'anomalie chromosomique) et en une seconde étape on peut par exemple en utilisant le cocktail n° 2 identifier la nature de la mutation des cellules mises en culture (dans ce cas, il s'agit du diagnostic des anomalies chromosomiques).

Le cocktail global a donc la composition suivante :

- héparine ............... de 150 à 400, notamment 200 µg/ml,
- γ-globuline humaine .... de 150 à 400, notamment 200 µg/ml,
- prostaglandine $E_2$ ....... de 0,8 à 10, notamment 1 µg/ml.

A titre d'exemple, on indique ci-après un protocole de traitement des cultures qui peuvent être mises en
oeuvre en vue de la réalisation des susdits dosages:

1°) Cultiver les cellules en flacon corning
75 cm$^2$, milieu HAM F10 (fibroblastes humains) jusqu'à ce
qu'il y ait inhibition de contact.

2°) Eliminer le milieu de culture, laver trois
fois avec une solution tamponnée pH 7,4, entre chaque lavage, laisser les cellules à 5 minutes en contact avec cette
solution.

3°) Préparer du milieu HAM F10 dépourvu de sérum
de veau foetal mais contenant 0,01 % d'albumine bovine
(Sigma Lab.).

4°) Eliminer la solution de lavage. Rincer une
fois avec le milieu défini sous 3°), puis remplir avec 5 ml
du même milieu.

5°) Incuber pendant 10-12 heures à 37°C, en
agitant très doucement.

6°) Renouveler le milieu et laisser pendant
trois jours toujours à 37°C et avec agitation.

7°) Effectuer un premier prélèvement de 100 µl
de milieu de culture à la fin du troisième jour (vers 19 h
environ) et un second prélèvement le lendemain matin (vers
9 h environ) .

Congeler immédiatement dans l'azote liquide les
aliquotes prélevées.

8°) Après le deuxième prélèvement, ajouter à la
culture cellulaire 500 µl du cocktail inducteur.

9°) A la fin de ce quatrième jour (toujours vers
19 h) et les matins suivants (cinquième jour et sixième
jour, vers 9 heures), prélever chaque fois des nouvelles
aliquotes de 100 µl à congeler immédiatement dans l'azote
liquide.

10°) Faire les dosages de l'enzyme choisie dans
tous les tubes en même temps (hexosaminidase) et comparer
les résultats entre les tubes contenant des cultures induites et les tubes contenant les cultures non induites.

11°) Le cas échéant, faire en parallèle les opérations 1°) à 10°) sur des cellules provenant d'individus témoins sains et comparer.

Dans ce qui précède les concentrations des différents constituants du cocktail inducteur sont respectivement sensiblement égales à dix fois les concentrations finales correspondantes désirées dans le milieu de culture final.

Les cultures de cellules qui, dans les séquences qui précèdent, répondent "positivement" à la composition inductrice peuvent alors être suspectées de porter l'une des anomalies recherchées.

Les cultures de cellules qui répondent "positivement" sont celles pour lesquelles les dosages de l'enzyme choisie font apparaître une production de l'enzyme étudiée beaucoup plus importante sur les aliquotes qui ont été prélevées au terme de l'étape 9°) ci-dessus que sur les aliquotes prélevées au terme de l'étape 7°) ci-dessus. Lorsque les mêmes opérations sont réalisées sur des cellules provenant d'individus témoins sains, une telle différence n'est pas observée.

Comme dans le cas de la première variante du procédé selon l'invention, on peut mettre à profit cette technique pour étudier les effets de médicaments à étudier sur une maladie déterminée, en associant des gammes de doses croissantes de ce médicament à la composition inductrice appropriée, qui est mise en contact avec la culture de cellules affectées de ladite maladie déterminée.

Selon une troisième variante enfin du procédé selon l'invention, notamment pour effectuer le diagnostic *in vitro* de maladies caractérisées par l'incapacité du sujet de synthétiser certaines enzymes spécifiques (par exemple une α-glucosidase dans la maladie de Pompe, une β-galactosidase dans le cas de la $gM_1$-gangliosidose, etc.), on réalise une culture des cellules à étudier en présence d'un dérivé stabilisé de l'AMP cyclique, qui soit de plus apte à traverser facilement les membranes cellulaires, et on recherche et on dose l'enzyme spécifique éventuellement produite. Le dosage de l'enzyme se fait soit dans le milieu intracellulaire, soit dans le milieu de culture.

Il apparaît en effet que ces dérivés de l'AMP cyclique, tout comme l'AMP cyclique naturel, conduisent à une induction coordonnée de la totalité des hydrolases susceptibles d'être produites par la cellule, à l'exclusion bien entendu de celles qu'elle n'est pas capable de synthétiser, d'où un procédé différentiel particulièrement efficace pour dépister le dernier type d'anomalies envisagées.

De tels dérivés d'AMP cyclique "stabilisés" sont disponibles dans le commerce : à titre d'exemple, on mentionne le complexe $N^6$, $D^{2'}$-dibutyryl-adénosyl 3',5'-cyclique, acide monophosphorique et le complexe 8-(4-chlorophénylthio)-adénosine-3',5' cyclique, monophosphate de sodium.

On peut notamment procéder selon le protocole expérimental de culture susmentionné. On règle avantageusement la concentration du dérivé de l'AMP cyclique à une concentration finale de $5.10^{-6}$ à $5.10^{-9}$ M/l, par exemple de $5.10^8$ M/l de milieu de culture, et on procède ensuite au dosage des enzymes concernées selon les méthodes, par exemple colorimétriques, déjà indiquées plus haut, en rapport avec la seconde variante du procédé de l'invention, plus particulièrement dans son application aux diagnostics des maladies lysozomiales, de la fibrose kystique ou de la dystrophie musculaire.

Cette troisième variante de procédé est avantageusement mise en oeuvre sur des cellules possédant une anomalie vraisemblable qui aura de préférence été révélée par l'application à des cultures cellulaires provenant du même individu de la première ou seconde variante de procédé susdéfini. L'anomalie spécifique sera alors plus particulièrement révélée par l'absence de production de l'enzyme déficiente (par exemple de l'α-glucosidase ou de la β-galactosidase). Cette déficience sera d'autant plus marquée que l'on observera une production maximum de toutes les autres enzymes étudiées.

Les cellules qui peuvent être mises en oeuvre dans chacun des procédés décrits dans ce qui précède peuvent être constituées par toutes cellules originaires d'un malade, d'un nouveau-né ou encore d'un embryon.

L'invention concerne enfin plus particulièrement le procédé d'induction visant à diagnostiquer le défaut génétique pouvant conduire à la fibrose kystique ou à la dystrophie musculaire, ce procédé consistant à produire des cultures des cellules provenant d'individus ou d'embryons étudiés, en présence de la composition d'induction formées par l'association héparine-globulines, notamment γ-globulines humaines, et à doser l'une au moins des enzymes induites dont les caractéristiques sont susceptibles de donner lieu aux différences de comportement permettant la différenciation des cellules atteintes de fibrose kystique et celles atteintes de dystrophie musculaire, dans les conditions qui ont été rappelées plus haut.

Comme il va de soi et comme il résulte d'ailleurs déjà de ce qui précède, l'invention ne se limite nullement à ceux de ses modes d'application et de réalisation qui ont été plus spécialement envisagés ; elle en embrasse au contraire toutes les variantes.

En particulier, l'invention concerne toutes les méthodes équivalentes permettant d'atteindre les mêmes buts. En particulier, on mentionnera parmi ces méthodes équivalentes celles mettant en jeu un dosage de toute enzyme ou autre constituant susceptible d'être synthétisé en proportions relativement plus importantes par les cellules, lorsque celles-ci sont affectées par l'une des maladies définies plus haut, que par des cellules originaires d'individus sains. En particulier, on peut avoir recours au dosage du cAMP qui se trouve lui-même synthétisé en quantités plus importantes par les cellules malades que par les cellules saines, dès qu'elles sont mises en contact avec tout inducteur du genre de ceux que les cellules malades ne sont plus capables de cataboliser ou, le cas échéant, avec un agent stimulant de l'adénylcyclase. En conséquence, le dosage du cAMP ou de tout autre composant synthétisé de façon globale en excès peut servir à la détection de l'existence ou de la potentialité de l'une des affections, en tout cas au niveau de la première étape mettant en jeu le susdit "cocktail global".

L'invention n'est pas davantage limitée à l'utilisation des constituants plus particulièrement nommés dans les exemples. Il en est particulièrement ainsi des agents de stimulation de l'adénylcyclase. Les hormones indiquées plus haut à titre d'exemple peuvent être remplacées par tout autre agent exerçant la même fonction, notamment par l'isoprotérénol. Celui-ci peut être utilisé en lieu et place des prostaglandines rappelées plus haut dans toutes les compositions qui ont été définies. Avantageusement, la teneur des solutions initiales en isoprotérénol sera telle que l'on puisse atteindre dans le milieu de culture final des concentrations de $10^{-6}$ M à $10^{-4}$ M, de préférence de l'ordre de $10^{-5}$ M, les concentrations relatives des autres constituants de ces solutions ou mélanges étant par ailleurs sensiblement les mêmes.

De même, on peut avoir recours à d'autres inhibiteurs de phosphodiestérases ou encore à d'autres inhibiteurs de la synthèse de protéines. L'expression "inhibiteurs de synthèse de protéines", telle qu'elle est définie dans la présente demande, s'étend aussi à tous composés capables de retarder ou de n'inhiber que temporairement la synthèse des protéines.

REVENDICATIONS

1 - Procédé de diagnostic *in vitro* de la potentialité ou de l'existence de la fibrose kystique ou de la dystrophie musculaire, caractérisé par le fait que l'on met en contact une culture de cellules provenant d'un individu ou d'un embryon étudié avec une composition d'induction contenant en association de l'héparine et des globulines, notamment des γ-globulines humaines, et en ce que l'on dose dans le milieu intracellulaire ou extracellulaire, au contact duquel se trouvaient lesdites cellules, le niveau d'activité de l'une des enzymes, notamment hydrolases, qui sont produites en excès chez des individus atteints de l'une des susdites maladies, vis-à-vis du niveau mesurable sur des cultures de cellules provenant d'individus sains.

2 - Procédé selon la revendication 1, caractérisé en ce que les proportions relatives des constituants de la composition d'induction vis-à-vis de la culture sont ajustées de façon à obtenir des concentrations finales :
- de 150 à 400, notamment de l'ordre de 200 μg d'héparine par ml de culture,
- de 150 à 400, notamment de l'ordre de 200 μg de γ-globulines par ml de culture.

3 - Procédé de diagnostic *in vitro* selon la revendication 1 de la potentialité ou de l'existence de la fibrose kystique, de la dystrophie musculaire ou d'au moins une autre maladie lysozomiale, caractérisé en ce que la susdite composition d'induction mise au contact de la culture de cellules contient en outre au moins un inducteur caractéristique de la susdite maladie lysozomiale et ayant pour effet d'induire un niveau d'activité excédentaire de l'enzyme en cause dans des cultures de cellules originaires d'un individu atteint de la susdite maladie lysozomiale.

4 - Procédé selon la revendication 3, caractérisé en ce que la proportion relative dudit inducteur est ajustée de façon à ce que la concentration finale de la culture en cet inducteur soit comprise entre 300 et 2.000 μg par ml de culture.

27

5 - Procédé selon l'une quelconque des revendications 1 à 4 de diagnostic *in vitro* de la potentialité ou de l'existence de la fibrose kystique, de la dystrophie musculaire ou d'une anomalie chromosomique ou, le cas échéant, d'une maladie lysozomiale, caractérisé en ce que la susdite composition mise au contact de la culture de cellules contient en outre une hormone stimulant l'adénylcyclase, telle qu'une prostaglandine, de préférence une prostaglandine $E_1$ ou $E_2$.

6 - Procédé selon la revendication 5, caractérisé en ce que les proportions relatives en prostaglandine, de préférence la prostaglandine $E_2$, sont ajustées vis-à-vis de la culture de façon à obtenir une concentration finale de 0,8 à 10 µg, notamment de l'ordre de 1 µg par ml de culture.

7 - Procédé selon la revendication 5 ou la revendication 6, caractérisé en ce que :
- dans une première phase, on met en contact une culture desdites cellules avec une composition d'induction ou "cocktail global" conforme à la revendication 5 ou 6 et, si l'activité de l'enzyme dosée atteint un niveau attestant de la présence de l'une des susdites maladies ou anomalies,
- dans l'une ou plusieurs phases supplémentaires, on met en contact des cultures de cellules provenant du même individu ou embryon avec des compositions ou "sous-cocktails" plus spécifiques contenant des inducteurs ou groupes d'inducteurs correspondant à des maladies ou groupes de maladies plus spécifiques, en vue de la répétition du susdit dosage.

8 - Procédé selon la revendication 7, caractérisé par le fait que l'enzyme étudiée est formée par l'hexosaminidase, l'α-mannosidase ou la phosphatase acide.

9 - Procédé selon la revendication 8, caractérisé en ce que, lorsque l'on a observé, au cours d'un précédent dosage, une production plus importante de l'enzyme étudiée,

28

telle que l'α-mannosidase ou la phosphatase acide, après mise en contact de ladite culture de cellules avec une composition d'induction ne contenant à titre d'inducteurs que l'héparine et les globulines, on soumet un milieu contenant l'enzyme au contact duquel avaient antérieurement été placées ou cultivées des cellules provenant du même individu à un traitement thermique, notamment à une température de l'ordre de 40,5°C lorsqu'il s'agit de l'α-mannosidase ou de 36,5°C lorsqu'il s'agit de la phosphatase acide, l'obtention d'une cinétique d'inactivation pouvant être corrélée à la fibrose kystique et l'absence de cinétique d'inactivation, tout au plus une cinétique d'inactivation réduite, pouvant alors être corrélée à une dystrophie musculaire.

10 - Procédé selon l'une quelconque des revendications 1 à 9 étendu à l'étude des effets d'un médicament à étudier sur la maladie ou l'anomalie révélable par l'inducteur ou le groupe d'inducteurs contenu dans la composition utilisée, caractérisé en ce que l'on procède à une série de cultures decellules en cause en présence, d'une part, de ladite composition d'induction et, d'autre part, de concentrations croissantes du médicament étudié, et en ce que l'on dose l'enzyme étudiée en présence de chacune des concentrations dudit médicament.

11 - Composition d'induction pour la mise en oeuvre du procédé selon l'une quelconque des revendications 1 à 10, caractérisée en ce qu'elle consiste en une solution contenant :
- de 1500 à 4000 μg, notamment de 2000 μg d'héparine par ml,
- de 1500 à 4000 μg, notamment de 2000 μg par ml de γ-globulines humaines.

12 - Composition selon la revendication 11, caractérisée en ce qu'elle contient en outre de 8 μg à 100 μg, notamment de 10 μg par ml de prostaglandine $E_2$.